# EUROPEAN PATENT APPLICATION

(11) **EP 2 098 259 A1**
(43) Date of publication of application: **09.09.2009**
(21) Application number: 09162640.8
(22) Date of filing: 05.04.2002
(51) Int. Cl.: A61M 5/46

(54) **Vaccine delivery device**

(30) Priority: 12.04.2001 GB 0109297
(62) Divisional of application: 02730103.5
(71) Applicant: GlaxoSmithKline Biologicals s.a., 1330 Rixensart (BE)
(72) Inventor: Dalton, Colin, Clive, B-1330, Rixensart (BE)
(74) Representative: Easeman, Richard Lewis

(57) **Abstract**

The present invention relates to novel methods of vaccination and vaccine delivery. In particular, the invention relates to methods of vaccination and pre-filled vaccine delivery devices designed to deliver the vaccine to the dermis of an individual.

## Description

The present invention relates to novel methods of vaccination and vaccine delivery. In particular, the invention relates to methods of vaccination and pre-filled vaccine delivery devices designed to deliver the vaccine into the dermis of an individual. The prefilled delivery devices of the present invention comprise a prefillable vaccine reservoir and a needle in fluid communication therewith, wherein the effective penetration depth of the needle into the skin of the individual is restricted by a limiter portion which engages with the surface of the skin such that the vaccine is delivered into the dermis of the skin. Specific vaccine formulations are also provided which are particularly potent in the induction of systemic immune responses. In particular, low dose and/or low volume vaccine formulations for delivery into the dermis are provided. Kits comprising a pre-filled syringe, and a needle-limiter attachment for assembly into the devices of the present invention are also provided. Also provided are methods of treating an individual susceptible or suffering from a disease, and methods of inducing an immune response comprising intradermal administration of a vaccine using a delivery device as described herein.

Vaccination of individuals has been performed by many routes of administration, the most common of which is administration of the vaccine into the deep muscle of the individual (intramuscular injection). Other well known routes of vaccination include sub-cutaneous, intranasal, oral, rectal and intraperitoneal and intradermal administration. Most of these routes of administration have always been associated with many drawbacks which are desirable to circumvent, including the pain associated with large needles, risk of infection in the injection site, and difficulty of administration.

In particular, intradermal administration of vaccines has been performed since early in the 20^{th} century with varying success. Most commonly this variability is associated with the difficulty in getting reproducible vaccine administration into the dermis. Commonly the administration of the vaccine is too deep into the skin causing sub-cutaneous or intramuscular administration, or too shallow, causing leakage of the vaccine out of the injection site.

The conventional technique of intradermal injection, the "mantoux procedure", is complex and requires a trained and skilled technicial to perform. The process comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

Delivery devices described for administration of agents into or across the skin include short needle devices such as those described in US 4,886,499, US5,190,521, US 5,328,483, US 5,527,288, US 4,270,537, US 5,015,235, US 5,141,496, US 5,417,662. Jet injection devices which deliver agents to the skin via a liquid jet injector or via a needle which pierces the stratum corneum and produces a jet which reaches the dermis are described for example in US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537.

It is desirable to provide an alternative way of administering vaccines, in particular a way that is pain-free or less painful than i.m. injection, and does not involve the associated negative affect on patient compliance because of "needle fear". It is also desirable to overcome the problems of mass population vaccination by intradermal vaccination, which until now have rendered the classical "mantoux" procedure inoperable for commercial vaccines. It would also be desirable to target the cell mediated immune system for example by targeting the antigen to the dendritic cells and langerhans cells that reside in the skin, particularly in the dermis. Cell mediated immunity appears to assist viral clearance and recovery from illness and may provide better cross protection between viral strains than antibodies. It has also been described in the literature that intradermal administration allows for the induction of a mucosal immunity at the level of the mucosal surfaces.

It has been found that these problems have been overcome, and additional advantages arise from using certain vaccine delivery devices and or specific vaccines. The vaccine delivery devices comprise in the first aspect of the present invention a device for controlling the penetration depth of a needle, for application to an injection syringe, **characterised in that** said device comprises a skin contacting element having a concave surface with respect to the skin, said skin contacting element encompassing, at least partially, the tip of said needle, and in that said skin contacting element is operatively associated with coupling means for connection with said syringe. In an embodiment of the invention described in WO 99/34850 there is described a device for controlling the penetration depth of a needle of an injection syringe which comprises a concave skin contacting element encompassing, at least partially, the tip of said needle, said skin contacting element being connected with said syringe, **characterised in that** said tip of said needle projects for a short length from said skin contacting element suitable for intradermal injection. In an alternate embodiment of the device there is provided a device for controlling the penetration depth of a needle of an injection syringe which comprises a concave skin contacting element, wherein the contoured surface of the skin contacting element sensitises the region encompassing the needle puncture region to reduce the pain of needle insertion.

Such devices are described in US 6,200,291 and its corresponding International application WO 99/34850. Neither US 6,200,291 nor its corresponding International application WO 99/34850, mention any specific uses of the device (which is currently sold for cosmetic treatment of cellulite, or "mesotherapy" - Di Pietro, 1997, Dermatologia Ambulatoriale, Anno V, Vol. 5, N.3, pages 25-27). These devices have been found by the present inventors to be exceptional in the induction of immunological responses after administering a vaccine into the dermis of a patient.

Accordingly in the first aspect of present invention the device described in US 6,200,291, the entire contents of which are incorporated herein by reference, is used for intradermal vaccination. There is also provided devices of US 6,200,291 which are pre-filled with a vaccine formulation, and methods of producing a vaccine comprising providing a vaccine formulation and filling it into a device as described in US 6,200,291. Also provided is a method of administering a vaccine, and a method of inducing an immune response, both of which comprise the administration of a vaccine into the dermis of a patient using a device described in US 6,200,291.

The vaccines of the present invention have been found to be so potent that they may be administered as "low dose" and/or "low volume" vaccine formulations.

In the context of this aspect of the present invention, when the device is that described in US 6,200,291, the vaccine may comprise an antigen which is selected from a human pathogen, such as a viral or bacterial antigen; or the antigen may be a human self-antigen for the treatment of a chronic disorder such as allergy, cancer, autoimmune disease, alzheimers and others.

Preferably the vaccine formulations which may be used in the first aspect of the present invention contain an antigen or antigenic composition capable of eliciting an immune response against a human pathogen, which antigen or antigenic composition is derived from HIV-1, (such as tat, nef, gp120 or gp160), human herpes viruses (HSV), such as gD or derivatives thereof or Immediate Early protein such as ICP27 from HSV1 or HSV2, cytomegalovirus (CMV (esp Human)(such as gB or derivatives thereof), Rotavirus (including live-attenuated viruses), Epstein Barr virus (such as gp350 or derivatives thereof), Varicella Zoster Virus (VZV, such as gpI, II and IE63), or from a hepatitis virus such as hepatitis B virus (for example Hepatitis B Surface antigen or a derivative thereof), hepatitis A virus (HAV), hepatitis C virus and hepatitis E virus, or from other viral pathogens, such as paramyxoviruses: Respiratory Syncytial virus (RSV, such as F and G proteins or derivatives thereof), parainfluenza virus, measles virus, mumps virus, human papilloma viruses (HPV, for example HPV6, 11, 16, 18), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as Neisseria spp, including N. gonorrhea and N. meningitidis (for example capsular polysaccharides and conjugates thereof, transfenin-binding proteins, lactoferrin binding proteins, PilC, adhesins); S. pyogenes (for example M proteins or fragments thereof, C5A protease, lipoteichoic acids), S. agalactiae, S. mutans; H. ducreyi; Moraxella spp, including M catarrhalis, also known as Branhamella catarrhalis (for example high and low molecular weight adhesins and invasins); Bordetella spp, including B. pertussis (for example pertactin, pertussis toxin or derivatives thereof, filamenteous hemagglutinin, adenylate cyclase, fimbriae), B. paraperlussis and B. bronchiseptica; Mycobacterium spp., including M. tuberculosis (for example ESAT6, Antigen 85A, -B or -C), M. bovis, M. leprae, M. avium, M. paratuberculosis, M. smegmatis; Legionella spp, including L. pneumophila; Escherichia spp, including enterotoxic E. coli (for example colonization factors, heat-labile toxin or derivatives thereof, heat-stable toxin or derivatives thereof), enterohemorragic E. coli, enteropathogenic E. coli (for example shiga toxin-like toxin or derivatives thereof); Vibrio spp, including V. cholera (for example cholera toxin or derivatives thereof); Shigella spp, including S. sonnei, S. dysenteriae, S. flexnerii; Yersinia spp, including Y. enterocolitica (for example a Yop protein), Y. pestis, Y. pseudotuberculosis; Campylobacter spp, including C. jejuni (for example toxins, adhesins and invasins) and C. coli; Salmonella spp, including S. typhi, S. paratyphi, S. choleraesuis, S. enteritidis; Listeria spp., including L. monocytogenes; Helicobacter spp, including H. pylori (for example urease, catalase, vacuolating toxin); Pseudomonas spp, including P. aeruginosa; Staphylococcus spp., including S. aureus, S. epidermidis; Enterococcus spp., including E. faecalis, E. faecium; Clostridium spp., including C. tetani (for example tetanus toxin and derivative thereof), C. botulinum (for example botulinum toxin and derivative thereof), C. difficile (for example clostridium toxins A or B and derivatives thereof); Bacillus spp., including B. anthracis (for example botulinum toxin and derivatives thereof); Corynebacterium spp., including C. diphtheriae (for example diphtheria toxin and derivatives thereof); Borrelia spp., including B. burgdorferi (for example OspA, OspC, DbpA, DbpB), B. garinii (for example OspA, OspC, DbpA, DbpB), B. afzelii (for example OspA, OspC, DbpA, DbpB), B. andersonii (for example OspA, OspC, DbpA, DbpB), B. hermsii; Ehrlichia spp., including E. equi and the agent of the Human Granulocytic Ehrlichiosis; Rickettsia spp, including R. rickettsii; Chlamydia spp., including C. trachomatis (for example MOMP, heparin-binding proteins), C. pneumoniae (for example MOMP, heparin-binding proteins), C. psittaci; Leptospira spp., including L. interrogans; Treponema spp., including T. pallidum (for example the rare outer membrane proteins), T. denticola, T. hyodysenteriae; or derived from parasites such as Plasmodium spp., including P. falciparum; Toxoplasma spp., including T. gondii (for example SAG2, SAG3, Tg34); Entamoeba spp., including E. histolytica; Babesia spp., including B. microti; Trypanosoma spp., including T. cruzi; Giardia spp., including G. lamblia; Leshmania spp., including L. major; Pneumocystis spp., including P. carinii; Trichomonas spp., including T. vaginalis; Schisostoma spp., including S. mansoni, or derived from yeast such as Candida spp., including C. albicans; Cryptococcus spp., including C. neoformans.

Other preferred specific antigens for *M. tuberculosis* are for example Tb Ra12, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748).

Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp*, including *S*. *pneumoniae* (for example capsular polysaccharides and conjugates thereof, PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Particularly preferred pneumococcal vaccines are those described in WO 00/56539. Other preferred bacterial vaccines comprise antigens derived from *Haemophilus spp*., including *H. influenzae type B* ("Hib", for example PRP and conjugates thereof), *non typeable H. influenzae*, for example OMP26, high molecular weight adhesins, P5, P6, protein D and lipoprotein D, and fimbrin and fimbrin derived peptides (US 5,843,464) or multiple copy varients or fusion proteins thereof

Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred aspect the vaccine formulation of the invention comprises the HIV-1 antigen, gp120, especially when expressed in CHO cells. In a further embodiment, the vaccine formulation of the invention comprises gD2t as hereinabove defined.

In a preferred embodiment of the present invention vaccines containing the claimed adjuvant comprise antigen derived from the Human Papilloma Virus (HPV) considered to be responsible for genital warts *(*HPV 6 or HPV 11 and others), and the HPV viruses responsible for cervical cancer (HPV16, HPV18 and others).

Particularly preferred forms of genital wart prophylactic, or therapeutic, vaccine comprise L1 particles or capsomers, and fusion proteins comprising one or more antigens selected from the HPV 6 and HPV 11 proteins E6, E7, L1, and L2.

The most preferred forms of fusion protein are: L2E7 as disclosed in WO 96/26277, and proteinD(1/3)-E7 disclosed in GB 9717953.5 (PCT/EP98/05285).

A preferred HPV cervical infection or cancer, prophylaxis or therapeutic vaccine, composition may comprise HPV 16 or 18 antigens. For example, L1 or L2 antigen monomers, or L1 or L2 antigens presented together as a virus like particle (VLP) or the L1 alone protein presented alone in a VLP or caposmer structure. Such antigens, virus like particles and capsomer are per se known. See for example WO94/00152, WO94/20137, WO94/05792, and WO93/02184.

Additional early proteins may be included alone or as fusion proteins such as E7, E2 or preferably E5 for example; particularly preferred embodiments of this includes a VLP comprising L1E7 fusion proteins (WO 96111272).

Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277).

Alternatively the HPV 16 or 18 early proteins E6 and E7, may be presented in a single molecule, preferably a Protein D- E6/E7 fusion. Such vaccine may optionally contain either or both E6 and E7 proteins from HBV 18, preferably in the form of a Protein D - E6 or Protein D - E7 fusion protein or Protein D E6/E7 fusion protein.

The vaccine of the present invention may additionally comprise antigens from other HPV strains, preferably from strains HPV 31 or 33.

Vaccines of the present invention further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The formulations may also contain an anti-tumour antigen and be useful for the immunotherapeutic treatment of cancers. The formulations may also contain an anti-tumour antigen and be useful for the immunotherapeutic treatment of cancers. For example, the adjuvant formulation finds utility with tumour rejection antigens such as those for prostrate, breast, colorectal, lung, pancreatic, renal or melanoma cancers. Exemplary antigens include MAGE 1 , 3 and MAGE 4 or other MAGE antigens such as disclosed in WO99/40188, PRAME, BAGE, Lage (also known as NY Eos 1) SAGE and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma. In a preferred embodiment prostate antigens are utilised, such as Prostate specific antigen (PSA), PAP, PSCA (PNAS 95(4) 1735 -1740 1998), PSMA or, in a preferred embodiment an antigen known as Prostase. Other tumour associated antigens useful in the context of the present invention include: Plu -1 J Biol. Chem 274 (22) 15633 -15645, 1999, HASH -1, HasH-2, Cripto (Salomon et al Bioessays 199, 21 61-70,US patent 5654140) Criptin US patent 5 981 215, ., Additionally, antigens particularly relevant for vaccines in the therapy of cancer also comprise tyrosinase and survivin. Mucin dervied peptides such as Muc1 see for example US 5744,144 US 5827, 666 WO 8805054, US 4,963,484. Specifically contemplated are Muc 1 derived peptides that comprise at least one repeat unit of the the Muc 1 peptide, preferably at least two such repeats and which is recognised by the SM3 antibody (US 6 054 438). Other mucin derived peptides include peptide from Muc 5.

The present invention is also useful in combination with breast cancer antigens such as her 2/ Neu, mammaglobin (US patent 5668267) or those disclosed in WO/00 52165, WO99/33869, WO99/19479, WO 98/45328. Her 2 neu antigens are disclosed inter alia, in US patent 5,801,005. Preferably the Her 2 neu comprises the entire extracellular domain ( comprising approximately amino acid 1 -645) or fragmants thereof and at least an immunogenic portion of or the entire intracellular domain approximately the C terminal 580 amino acids . In particular, the intracellular portion should comprise the phosphorylation domain or fragments thereof. Such constructs are disclosed in WO00/44899.

Vaccines of the present invention may be used for the prophylaxis or therapy of allergy. Such vaccines would comprise allergen specific (for example Der p1) and allergen non-specific antigens (for example peptides derived from human IgE, including but not restricted to the stanworth decapeptide (EP 0 477 231 B1)).

Vaccines of the present invention may also be used for the prophylaxis or therapy of chronic disorders others than allergy, cancer or infectious diseases. Such chronic disorders are diseases such as atherosclerosis, and Alzheimer.

Antigens relevant for the prophylaxis and the therapy of patients susceptible to or suffering from Alzheimer neurodegenerative disease are, in particular, the N terminal 39 -43 amino acid fragment (Aβ of the amyloid precursor protein and smaller fragments. This antigen is disclosed in the International Patent Application No. WO 99/27944 - (Athena Neurosciences).

The most preferred antigens for use in the first aspect of the present invention are selecting from the group consisting of RSV, Streptococcus (and in particular using the vaccines described in WO 00/56359 the contents of which are incorporated herein by reference), HSV, HAV, HBV, VZV, HPV, and CMV.

In addition, at least two of the vaccines in this most preferred restricted list may be combined to form preferred vaccine combinations; for example the combination of a Streptococcus and RSV vaccine, and a combination of an HPV and HSV vaccine, and a combination of an HBV and HAV vaccine. Another specific vaccine combination that may be used in this second aspect of the present invention include the Infanrix™ range, made by GlaxoSmithKline Biologicals. Such vaccines are based on a "core" combination of Diptheria toxin, Tetanus toxin, and *B. pertussis* antigens. This vaccine comprises a pertussis component (either killed whole cell *B. pertussis* or accellular *pertussis* which typically consists of two antigens - PT and FHA, and often 69kDa, optionally with one or both agglutinogen 2 or agglutinogen 3). Such vaccines are often referred to as DTPw (whole cell) or DTPa (acellular).

Particular combination vaccines within the scope of the invention include:
Diptheria-Tetanus- Pertussis-Hepatitis B (DTP-HB)
Diptheria-Tetanus-Hepatitis B (DT-HB)
Hib-Hepatitis B
DTP-Hib-Hepatitis B
IPV (inactivated polio vaccine)- DTP-Hib-Hepatitis B [e.g. Infanrix-Hexa TM
- SmithKline Beecham Biologicals s.a.]
Diptheria-Tetanus- Pertussis-Hepatitis B-IPV (DTP-HB-IPV) [e.g. Infanrix-
Penta TM - SmithKline Beecham Biologicals s.a.].

The pertussis component is suitably a whole cell pertussis vaccine or an acellular pertussis vaccine containing partially or highly purified antigens. The above combinations may optionally include a component which is protective against Hepatitis A. Preferably the Hepatitis A component is formalin HM-175 inactivated. Advantageously, the HM-175 is purified by treating the cultured HM-175 with trypsin, separating the intact virus from small protease digested protein by permeation chromatography and inactivating with formalin. Advantageously the Hepatitis B containing combination vaccine is a paediatric vaccine.

In the second aspect of the present invention, the device used to administer the vaccine formulation may be that described in US 6,200,291 and also it may be any "similar" device, together with a vaccine formulation that is within a particular preferred list of vaccine preparations. Accordingly, in the second aspect of the present invention there is provided an intradermal vaccine, and a method of delivering a vaccine, wherein the vaccine antigen is selecting from the group consisting of RSV, Streptococcus (and preferably the vaccines described in WO 00/56359 the contents of which are incorporated herein by reference), HSV, HAV, HBV, VZV, HPV, and CMV. In addition, at least two of the vaccines in this restricted list may be combined to form preferred vaccine combinations; for example the combination of a Streptococcus and RSV vaccine, and a combination of an HPV and HSV vaccine, and a combination of an HBV and HAV vaccine. Another specific vaccine combination that may be used in this second aspect of the present invention include the Infanrix™ range, made by GlaxoSmithKline Biologicals. Such vaccines are based on a "core" combination of Diptheria toxin, Tetanus toxin, and *B. pertussis* antigens. This vaccine comprises a pertussis component (either killed whole cell *B. pertussis* or acellular *pertussis* which typically consists of two antigens - PT and FHA, and often 69kDa, optionally with one or both agglutinogen 2 or agglutinogen 3). Such vaccines are often referred to as DTPw (whole cell) or DTPa (acellular).

Particular combination vaccines within the scope of the invention include:
Diptheria-Tetanus- Pertussis-Hepatitis B (DTP-HB)
Diptheria-Tetanus-Hepatitis B (DT-HB)
Hib-Hepatitis B
DTP-Hib-Hepatitis B
IPV (inactivated polio vaccine)- DTP-Hib-Hepatitis B [e.g. Infanrix-Hexa TM
- SmithKline Beecham Biologicals s.a.]
Diptheria-Tetanus- Pertussis-Hepatitis B-IPV (DTP-HB-IPV) [e.g. Infanrix-
Penta TM - SmithKline Beecham Biologicals s.a.].

The pertussis component is suitably a whole cell pertussis vaccine or an acellular pertussis vaccine containing partially or highly purified antigens. The above combinations may optionally include a component which is protective against Hepatitis A. Preferably the Hepatitis A component is formalin HM-175 inactivated. Advantageously, the HM-175 is purified by treating the cultured HM-175 with trypsin, separating the intact virus from small protease digested protein by permeation chromatography and inactivating with formalin. Advantageously the Hepatitis B containing combination vaccine is a paediatric vaccine.

In this second aspect of the invention, devices that are "similar" to that described in US 6,200,291 are those that share the essential characteristics of US 6,200,291 that enable it to inject a vaccine efficiently and reproducibly into the dermis of a patient. Accordingly, "similar" devices comprise a conventional needle, which is attachable onto a syringe body, the device also having a skin contacting limiter member which is in an orientation with respect to the needle that in use the needle penetration into the skin of a patient is limited so that the vaccine is delivered into the dermis of the patient. Specific "similar" devices that are encompassed in this context are those described in JP 2000 - 37456 which discloses a vaccination device comprising a syringe and conventional needle, the syringe also having a cylindrical element extending from the syringe body which effectively limits the needle penetration depth to the sub-cutaneous tissue of the vaccinee. Accordingly the devices described in JP 2000-37456 wherein the needle extends beyond the puncture adjusting device by between about 1.0 and about 2.0 mm, and more preferably about 1.5 mm are similar devices to those described in US 6,200,291.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis. Accordingly, in the context of both the first and second aspects of the present invention, and skin-contacting limiter portions that shorten the effective length of a needle (such as the devices described in US 6,200,291 and JP 2000 37456), the needle in general extends beyond the skin contacting surface by a length of between about 1.0 and 2.0 mm, and preferably by about 1.5mm.

Preferably the volume of a dose of vaccine according to either aspect of the invention is between 0.025 ml and 2.5 ml, more preferably in a range of between approximately 0.1 ml and approximately 0.2 ml. A 50 µl dose volume might also be considered. A 0.1 ml dose is approximately one fifth of the volume of a conventional intramuscular vaccine dose. The volume of liquid that can be administered intradermally depends in part upon the site of the injection. For example, for an injection in the deltoid region, 0.1 ml is the maximum preferred volume whereas in the lumbar region a large volume e.g. about 0.2 ml can be given.

Preferably the vaccines according to both aspects of the invention are administered to a location between about 1.0 and 2.0 mm below the surface of the skin. More preferably the vaccine is delivered to a distance of about 1.5 mm below the surface of the skin.

The content of antigens in the intradermal vaccines of both aspects of the present invention may be similar to conventional doses as found in intramuscular vaccines. Accordingly, the protein antigens present in the intradermal vaccines may be a "high dose" vaccine in the range 1-100µg, preferably 5-50µg. Likewise, if present, the amount of polysaccharide conjugate antigen in each vaccine dose is generally expected to comprise 0.1-100 µg of polysaccharide, preferably 0.1-50 µg, preferably 0.1-10 µg, and may be between 1 and 5 µg. However, the most preferred vaccines of the present invention are "low dose" vaccines and may comprise as little as 1/5^{th} or even 1/10^{th} of the conventional intramuscular dose. Accordingly the protein antigens are preferably present in as little as 0.1 to 10µg, preferably 0.1 to 5 µg per dose; and if present the polysaccharide conjugate antigens may be present in the range of 0.01-1µg, and preferably between 0.01 to 0.5 µg of polysaccharide per dose.

In both aspects of the present invention it is preferred that the vaccine delivery devices and methods of vaccination are "booster" vaccines. That is to say that the vaccines are given to individuals that are immunologically primed to the specific vaccine antigen either by previous vaccination with that antigen by another route (such as intramuscular injection) or primed by previous infection with the pathogen that comprises that antigen. Most preferably the vaccination method comprises priming an individual by an intramuscular administration of a full dose vaccine, followed by an intradermal booster using a low dose vaccine administered using an intradermal administration device as described herein.

The vaccines according to either aspect of the present invention may further comprise an adjuvant or immunostimulant. It has long been known that enterobacterial lipopolysaccharide (LPS) is a potent stimulator of the immune system, although its use in adjuvants has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419) and has the following structure:

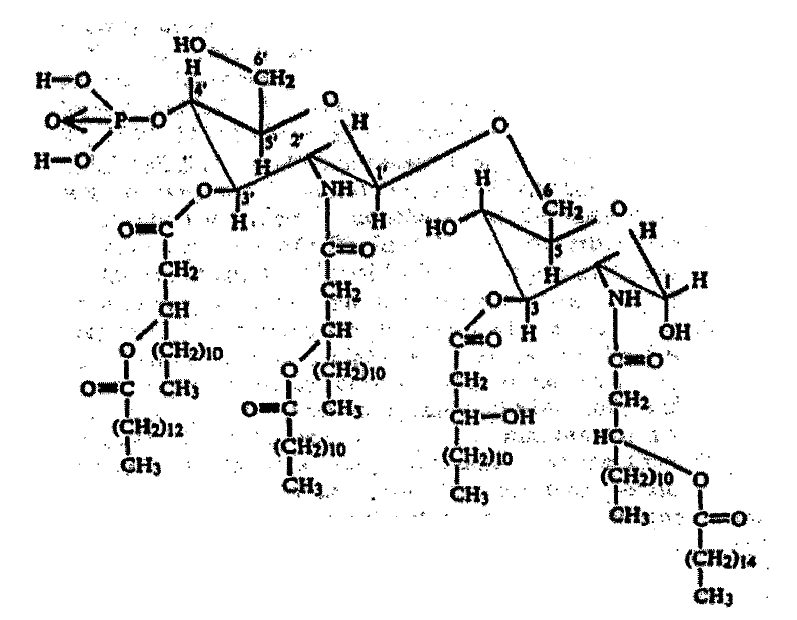

A further detoxified version of MPL results from the removal of the acyl chain from the 3-position of the disaccharide backbone, and is called 3-O-Deacylated monophosphoryl lipid A (3D-MPL). It can be purified and prepared by the methods taught in GB 2122204B, which reference also discloses the preparation of diphosphoryl lipid A, and 3-O-deacylated variants thereof.

A preferred form of 3D-MPL is in the form of an emulsion having a small particle size less than 0.2µm in diameter, and its method of manufacture is disclosed in WO 94/21292. Aqueous formulations comprising monophosphoryl lipid A and a surfactant have been described in WO9843670A2.

The bacterial lipopolysaccharide derived adjuvants to be formulated in the compositions of the present invention may be purified and processed from bacterial sources, or alternatively they may be synthetic. For example, purified monophosphoryl lipid A is described in Ribi et al 1986 (supra), and 3-O-Deacylated monophosphoryl or diphosphoryl lipid A derived from *Salmonella sp*. is described in GB 2220211 and US 4912094. Other purified and synthetic lipopolysaccharides have been described (Hilgers et al., 1986, Int.Arch.Allergy.Immunol., 79(4):392-6; Hilgers et al., 1987, Immunology, 60(1):141-6; and EP 0 549 074 B1). A particularly preferred bacterial lipopolysaccharide adjuvant is 3D-MPL.

Accordingly, the LPS derivatives that may be used in either aspect of the present invention are those immunostimulants that are similar in structure to that of LPS or MPL or 3D-MPL. In another aspect of the present invention the LPS derivatives may be an acylated monosaccharide, which is a sub-portion to the above structure of MPL.

A preferred disaccharide LPS derivative adjuvant, is a purified or synthetic lipid A of the following formula: 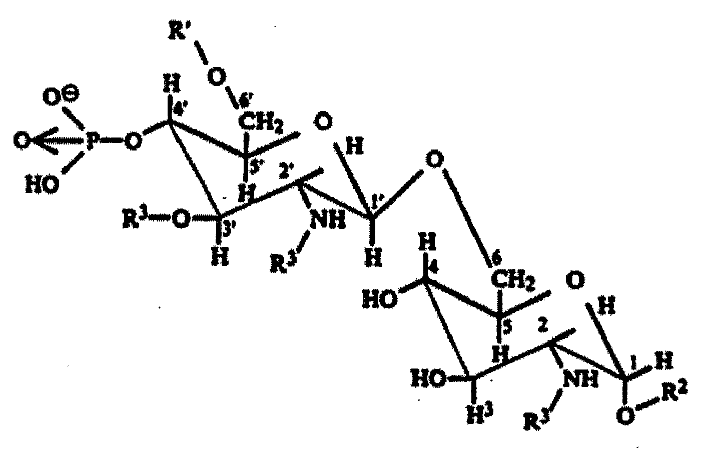 wherein R2 may be H or PO3H2; R3 may be an acyl chain or β-hydroxymyristoyl or a 3-acyloxyacyl residue having the formula:
wherein R⁴ 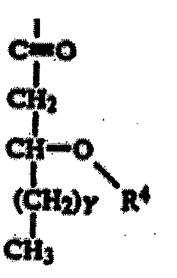 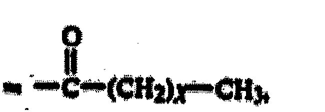 and wherein X and Y have a value of from 0 up to about 20.

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1. Particulate structures, termed Immune Stimulating Complexes (ISCOMS), comprising fractions of Quil A are haemolytic and have been used in the manufacture of vaccines (Morein, B., EP 0 109 942 B1; WO 96/11711; WO 96/33739). The haemolytic saponins QS21 and QS 17 (HPLC purified fractions of Quil A) have been described as potent systemic adjuvants, and the method of their production is disclosed in US Patent No.5,057,540 and EP 0 362 279 B1. Other saponins which have been used in systemic vaccination studies include those derived from other plant species such as Gypsophila and Saponaria (Bomford et al., Vaccine, 10(9):572-577, 1992).

An enhanced system involves the combination of a non-toxic lipid A derivative and a saponin derivative particularly the combination of QS21 and 3D-MPL as disclosed in WO 94/00153, or a less reactogenic composition where the QS21 is quenched with cholesterol as disclosed in WO 96/33739.

A particularly potent adjuvant formulation involving QS21 and 3D-MPL in an oil in water emulsion is described in WO 95/17210 and is a preferred formulation.

In one preferrred embodiment of either aspect of the present invention, the intradermal vaccines comprise a vesicular adjuvant formulation comprising cholesterol, a saponin and an LPS derivative. In this regard the preferred adjuvant formulation comprises a unilamellar vesicle comprising cholesterol, having a lipid bilayer preferably comprising dioleoyl phosphatidyl choline, wherein the saponin and the LPS derivative are associated with, or embedded within, the lipid bilayer. More preferably, these adjuvant formulations comprise QS21 as the saponin, and 3D-MPL as the LPS derivative, wherein the ratio of QS21:cholesterol is from 1:1 to 1:100 weight/weight, and most preferably 1:5 weight/weight. Such adjuvant formulations are described in EP 0 822 831 B, the disclosure of which is incorporated herein by reference.

Both aspects of the present invention provide a pharmaceutical kit comprising an intradermal administration device and a vaccine formulation as described herein. The device is preferably supplied already filled with the vaccine. Preferably the vaccine is in a liquid volume smaller than for conventional intramuscular vaccines as described herein, particularly a volume of between about 0.05 ml and 0.2 ml. Preferably the device is a short needle delivery device for administering the vaccine to the dermis.

Also provided by both aspects of the present invention are methods of inducing an immune response and methods of treating an individual susceptible to or suffering from a disease by intradermal administration of a vaccine as described herein.

### Example 1- Hepatitis B ID vaccination

A human clinical trial was carried out on human subjects to assess the benefits of ID delivery with a hepatitis B vaccine.

Patients were injected with either GSK Engerix™ vaccine (Hepatitis B vaccine) comprising either 2 or 20 µg of HbsAg. A standard formulation of Engerix™ comprises 20 µg of HbsAg per 1 ml. To deliver 2 µg of antigen 100 µl of the vaccine was used. Engerix™-B was supplied as pre-filled syringes (PFS) containing per 1.0 ml for the full dose IM administration

| | |
|---|---|
| Hepatitis B (recombinant HBsAg): | 20 µg |
| Aluminium salt: | 0.5 mg |

### Delivery route

Intradermal (ID) delivery was carried out using a device as disclosed in EP 1092444, the whole contents of which are herein incorporated by reference, wherein the device has a flat skin contacting element that effectively limits the penetration depth of the needle into the dermis. This is a "similar device" to that described in US6,200,291, and as such is part of the second aspect of the present invention. Effective needle length was approximately 1.5 mm.

Specifically a conventional tuberculine syringe was filled with approximately 150 µl of vaccine solution taken from a 0.5 ml vial. A standard needle was used for this procedure. Then, the standard needle was discarded and replaced on the tuberculine syringe with an intradermal needle equipped with a skin penetration limiter, as detailed in EP 1092444. Any air bubbles were eliminated from the syringe and the filling volume reduced to 100 µl (which corresponds to an antigen dose of 2 µg). The syringe was placed perpendicular to the skin (at a 90° angle). The needle was introduced firmly into the skin until the skin came in close contact with the penetration limiter. A light pressure was maintained during vaccine injection to allow continuous contact of the penetration limiter with the skin to ensure correct intradermal administration and to limit possible vaccine leakage from the injection site.

IM delivery was carried out using a standard needle. For a full (20 µg) dose, the doses were administered as deep intramuscular injection in the deltoid muscle of the non-dominant arm (using a PFS with a 23-gauge needle). For a fraction (2 µg) dose the doses were administered as deep intramuscular injection in the deltoid muscle of the non-dominant arm (using a tuberculine syringe with a 23-gauge needle).

5 groups of individuals were tested in 5 parallel groups. There were 175 subjects (35 per group). The study tested healthy adults aged between 18 and 45 years

### Vaccination regime

Vaccination was carried out as follows:

| | |
|---|---|
| Group 1 | Initially anti-HBs seronegative; 20 µg intramuscularly at months 0, 1, 6 |
| Group 2 | Initially anti-HBs seronegative; 2 µg intramuscularly at months 0, 1, 6 |
| Group 3 | Initially anti-HBs seronegative; 2 µg intradermally in the deltoid region at months 0, 1, 6 |
| Group 4 | Initially anti-HBs seropositive; 2 µg intradermally in the deltoid region at month 0 |
| Group 5 | Initially anti-HBs seropositive; 20 µg intramuscularly at month 0 |

For groups 1-3, samples were taken at 1 month (post I), 2 months (post 2) and 7 months (post III). For groups 4 and 5 vaccination was carried out at month 0, and samples for analysis post boosting taken at 1 month.

### Results

| | | Primary vaccination Booster | | | | |
|---|---|---|---|---|---|---|
| | | Group 1 20 µg IM | Group 2 2 µg IM | Group 3 2 µg ID | Group 4 2 µg ID | Group 5 20 µg IM |
| | *n* | *32* | *33* | *33* | *32* | *30* |
| PI | S+ (%) | 6.3 | 0 | 6.1 | | |
| | SP (%) | 3.1 | 0 | 0 | | |
| | GMT | 7.1 | | 5.1 | | |
| PII | S+ (%) | 59.4 | 9.1 | 24.2 | | |
| | SP (%) | 46.9 | 9.1 | 12.1 | | |
| | GMT | 33.4 | 88 | 10.1 | | |
| PIII | S+ (%) | 93.8 | 48.5 | 80.6 | | |
| | SP (%) | 90.6 | 48.5 | 74.2 | | |
| | GMT | 6910.8 | 441.2 | 328 | | |
| Pre-booster | S+ (%) | | | | 59.4 | 63.3 |
| | SP (%) | | | | 50 | 50 |
| | GMT | | | | 28 | 23 |
| Post-booster | S+ (%) | | | | 96.9 | 93.5 |
| | SP (%) | | | | 93.8 | 93.5 |
| | GMT | | | | 1272 | 7789.8 |

| | | | | | | |
|---|---|---|---|---|---|---|
| N = number of vaccinees PI = post first injection (1 month) PII = post second injection (2 months) PIII= post third injection (7months) S+% = % seropositive individuals SP% = % seroprotection GMT = geometric mean titre EL.U/ml | | | | | | |

### Conclusions

The results indicate that patients vaccinated ID for primary vaccination have equivalent or slightly higher % seroprotection and % seropositivity than those patients treated with the equivalent volume of antigen IM. These results support the approach of hepB ID delivery in combination with a specific ID delivery device as exemplified herein.

In boosting, the use of 2 µg HbsAg ID provides at least equivalent % seroprotection and % seropositivity than 20 µg HbsAg IM.

## Claims

1. Use of a device for application to an injection syringe having a needle, **characterised in that** said device comprises a concave skin contacting element encompassing, at least partially, the tip of said needle, and **in that** when applied to said injection syringe the needle projects from said skin contacting element for a short length suitable for intradermal injection, for administering a vaccine.

2. A prefilled vaccine delivery device comprising a vaccination device for controlling the penetration depth of a needle, for application to an injection syringe, **characterised in that** said device comprises a concave skin contacting element encompassing, at least partially, the tip of said needle, and **in that** said skin contacting element is operatively associated with coupling means for connection with said syringe, and a vaccine reservoir containing a vaccine formulation.

3. A prefilled vaccine delivery device as claimed in claim 2 wherein the needle of said delivery device projects from said skin contacting element for a short length suitable for intradermal injection.

4. A prefilled vaccine delivery device as claimed in claim 2 or 3 wherein the vaccine is a low volume vaccine of between approximately 0.1 to approximately 0.2 ml in volume.

5. A prefilled vaccine delivery device as claimed in claim 4 wherein the vaccine is a low dose vaccine comprising between 0.1 and 10 µg of protein antigen, or between 0.01 and 1µg of polysaccharide antigen.

6. A method of vaccination comprising administering a vaccine formulation into the dermis of a patient using a prefilled vaccine delivery device as claimed in any one of claims 2 to 5.

7. A prefilled vaccine delivery device comprising a needle, which is attachable onto a syringe body filled with a vaccine formulation comprising an antigen selected from the group consisting of RSV, Streptococcus, HSV, HAV, HBV, VZV, HPV, and CMV, the device having a skin contacting element which is in an orientation with respect to the needle that in use the needle penetration into the skin of a patient is limited so that the vaccine is delivered into the dermis of the patient.

8. A prefilled vaccine delivery device as claimed in claim 7 wherein the vaccine is a low volume vaccine of between approximately 0.1 to approximately 0.2 ml in volume.

9. A prefilled vaccine delivery device as claimed in claim 8 wherein the vaccine is a low dose vaccine comprising between 0.1 and 10 µg of protein antigen, or between 0.01 and 1µg of polysaccharide antigen.

10. A method of vaccination comprising administering a vaccine formulation comprising a vaccine antigen selected from the group consisting of RSV, Streptococcus, HSV, HAV, HBV, VZV, HPV, and CMV wherein the method comprises administering the vaccine to the dermis of the skin using a vaccine delivery device which has a needle, which is attachable onto a syringe body filled with a vaccine formulation, the device having a skin contacting element which is in an orientation with respect to the needle that in use the needle penetration into the skin of a patient is limited so that the vaccine is delivered into the dermis of the patient.
